# EUROPÄISCHE PATENTSCHRIFT

(11) **EP 2 934 348 B1**
(45) Veröffentlichungstag und Bekanntmachung des Hinweises auf die Patenterteilung: **21.03.2018**
(21) Anmeldenummer: 13818205.0
(22) Anmeldetag: 13.12.2013
(51) Int. Cl.: A61B 17/12, A61F 13/02, A61B 17/00, A61B 17/132, A61F 13/00, A61B 90/00

(54) **GEFÄSSVERSCHLUSSSYSTEM**
VESSEL CLOSURE SYSTEM
SYSTÈME DE FERMETURE DE RÉCIPIENT

(30) Priorität: 21.12.2012 EP 12405128
(43) Veröffentlichungstag der Anmeldung: 28.10.2015
(73) Patentinhaber: VOSTRA-MED AG, 6330 Cham (CH)
(72) Erfinder: HARREN, Ernst-Diethelm, CH-6343 Rotkreuz (CH); ACKERMANN, Simon, CH-8442 Hettlingen (CH)
(74) Vertreter: Luchs, Willi
(86) Internationale Anmeldenummer: PCT/EP2013/076570
(87) Internationale Veröffentlichungsnummer: WO 2014/095646

(56) Entgegenhaltungen:
- EP-A1- 2 404 549
- DE-U1-202011 106 809
- US-A- 4 592 342
- US-A- 5 269 803
- US-A- 5 312 350
- US-A- 5 376 067

## Beschreibung

Die Erfindung betrifft ein Gefässverschlusssystem nach dem Oberbegriff des Anspruchs 1.

Insbesondere betrifft die Erfindung ein Gefässverschlusssystem zur Sicherung der Punktionsstelle in der Haut und der Einstichstelle eröffneter Gefässe vor Nachblutungen, und welches der permanenten visuellen Überprüfung eines möglichen Blutaustrittes dient. Ebenso soll mit diesem eine ertastbare und akustische Kontrolle eines ungehinderten Blutflusses im Gefäss erzielt werden.

Die Erfindung betrifft insbesondere ein ortsdefiniert aufklebbares Gefässverschlusssystem zur Sicherung eröffneter Gefässe vor Nachblutungen mit mindestens einem Druckkörper, der geeignet ist, mit einer Wunde in Berührung zu gelangen.

Gefässverschlusssysteme sind wirksam in Bezug auf die Zeit bis zur Blutstillung. In diesem Bereich der medizinischen Anwendungen, insbesondere aber bei der Eröffnung von Gefässen für interventionelle Eingriffe, gibt es noch immer einen Bedarf, eine auch für medizinisches Fachpersonal einfach zu handhabende Sicherung eröffneter Gefässe zur Verfügung zu stellen, mit der nicht nur ein späterer Blutaustriff bzw. Blutverlust in den freien Raum ausserhalb des Körpers, sondern auch der Blutaustritt in das Gewebe (Hämatombildung) verhindert wird, wobei gleichzeitig aber auch die lebenswichtige Blutzirkulation innerhalb des Körpers nach erfolgtem Einsatz und endgültigem Anlegen des Gefässverschlusssystems nachweisbar nicht behindert werden soll.

In der Druckschrift US 5,376,067 ist eine Druckbandage offenbart, bei welcher bei einer Variante Bänder vorgesehen sind, welche mit Klebebeschichtungen versehen und auf die Haut klebbar sind. Es ist ein Ballon und eine über diesen gelegte sterile Gaze vorhanden. Bei einer andern Ausführung sind ähnliche Druckbandagen mit einem länglicheren Ballon für das Anlegen an einen Oberschenkel vorgesehen. Dieser Ballon ist dabei von einer Klebeschicht umgeben und zudem sind quer zu diesem verlaufende Pflaster rückseitig des Ballons angeordnet.

In herkömmlicher Weise geschieht das Verschliessen eröffneter Gefässe derart, dass nach dem Eingriff an der Punktionsstelle und der Gefässeinstichstelle gleichzeitig im richtigen Mass manueller Druck ausgeübt wird. Wegen der Gefahr der Hömatombildung darf der Druck nicht zu klein sein, wegen der Gefahr der Kreislaufunterbindung im betroffenen Körperteil aber auch nicht zu gross.

Nachdem eine Blutstillung erreicht ist, werden mechanische Hilfsmittel derart mit Verbänden am Körper befestigt, dass das mechanische Hilfsmittel in Verbindung mit dem Verband die weitere Kompression übernimmt.

Es ist ausserdem eine Verschlussvorrichtung gemäss DE-20 2011 106 089 U1 bekannt, die die Merkmale des Oberbegriffs von Anspruch 1 offenbart und bei welcher eröffnete Blutgefässe verschliessbar sind, bei der eine Vielzahl von Haltebändern aus einem unelastischen Material und mit diesen verbundenes Verschlusselement vorgesehen sind. Dabei ist zumindest ein Indikatorelement vorhanden durch welches die Streckung der Zugkraft des Verschlusselementes erkennbar ist. Es ist Aufgabe der vorliegenden Erfindung ein komplikationsfreies Gefässverschlusssystem aufzuzeigen.

Das erfindungsgemässe Gefässverschlusssystem gemäss Anspruch 1 gestattet es überraschend einfach, einen individuell durch Zugkraft einstellbaren Druck auf die Gefässöffnung anzulegen. Ferner ist auf einfache Weise ein Gefässverschlusssystem geschaffen, bei welchem eine unterschiedliche Verschlusselementgeometrie zur Verfügung gestellt ist. Im Gegensatz zu Verschlusssystemen aus dem Stand der Technik kann somit berücksichtigt werden, dass sich die Punktionsstelle auf der Haut und die Gefässeinstichstelle auf unterschiedlichen Ebenen befinden. Damit ergibt sich eine einwandfreies Verschliessen dieser Einstichstellen.

Bei Notwendigkeit kann durch ein Drücken auf der Rückseite des Druckkörpers eine temporäre, unterstützende Kompression vorgenommen werden. Beim Verlassen der medizinischen Einrichtung können die Patienten das Gefässverschlusssystem auf der Einstichstelle belassen.

Vorzugsweise ist der wenigstens eine Druckkörper derart ausgelegt, dass mit ihm ein in Relation zur Einstichstelle im Gefäss reduzierter Druck an einen Bereich zur Pulsmessung am Gefäss angelegt wird. Dadurch kann das Gefässverschlusssystem mit einem Pulsmesssensor erweitert werden, der gegebenenfalls Blutstauungen detektiert, bevor diese auftreten. Der transparente Druckkörper lässt die Beobachtung der Punktionsstelle uneingeschränkt zu. Ebenfalls besteht durch Anschliessen eines Blutdruckmessgeräts die Möglichkeit, per Messfühler den ungehinderten Blutfluss im Gefäss permanent zu überwachen.

Vorzugsweise ist der wenigstens eine Druckkörper der anatomischen Form einer Fingerabbildung zum manuellen Verschliessen der Punktionsstelle in der Haut bzw. des eingestochenen Gefässes nachgebildet. Der Einsatz eines solchen Gefässverschlusssystems kann auf verschiedene Weise erfolgen. Entweder kann das Gefäßverschlusssystem als Primärverschluss, d.h. das Gefässverschlusssystem wird unmittelbar nach dem invasiven Eingriff aufgebracht, oder als Sekundärverschlusssystem eingesetzt, bei welcher der behandelnde Arzt nach dem invasiven Eingriff die Punktionsstelle in der Haut und die Gefässeinstichstelle solange gleichzeitig manuell abdrückt, bis die Blutflussstillung erreicht ist. Hiernach platziert der behandelnde Arzt das Gefässverschlusssystem derart, dass sowohl die Punktionsstelle in der Haut als auch die Gefässeinstichstelle zur Abdeckung kommen.

Im Rahmen der Erfindung entspricht die anatomische Form des Druckkörpers einer Nachbildung von drei Fingern, vorzugsweise mit dem mittleren Finger.

In einer besonderen Ausführungsform ist der Druckkörper in Längserstreckung der Haltebänder angeordnet.

Das Gefässverschlusssystem kann ferner eine Vielzahl von Haltebändern aufweisen, welche an distalen Enden des Verschlusselementes anschliessen, wobei die Haltebänder unelastische Eigenschaften haben.

Vorzugsweise sind das Verschlusselement und die Haltebänder einstückig ausgebildet. Das Gefässverschlusssystem ist hierbei mit geeignet langen Haltebänder versehen. Neben der guten Handhabbarkeit soll damit auch eine genügende Haftung auf der Haut erreicht werden, um den zur Blutstillung nötigen Gegendruck aufbauen zu können.

Vorzugsweise ist jeweils in dem Bereich der Haltebänder, auf einer Seite des Gefässverschlusssystems, auf welcher der wenigstens eine Druckkörper bereitgestellt ist, eine Verstärkungsschicht angebracht, welche aus einem Material mit unelastischen Eigenschaften ausgebildet ist. Mittels dieser Verstärkungsschicht werden den Haltebändern die notwendigen unelastischen Eigenschaften vermittelt.

Vorteilhaft ist die Verstärkungsschicht mittels eines Klebemittels jeweils an einer Seite mit dem Bereich der Haltebänder verbunden und auf der weiteren Seite mit einer Hautkleberschicht bereitgestellt. Mit Hilfe der Hautkleberschicht wird das Gefässverschlusssystem derart an der Haut des Patienten fixiert, dass der Druckkörper die Punktionsöffnung hierdurch zuverlässig verschliesst.

Die Hautkleberschicht kann mit einer abziehbaren Schutzfolie bedeckt werden. Hierdurch ist die Hautkleberschicht geschützt und somit eine langandauernde Klebekraft der Hautkleberschicht gewährleistet.

Vorzugsweise sind das Verschlusselement und/oder der wenigstens eine Druckkörper und/oder die Haltebänder und/oder die Verstärkungsschicht transparent oder annähernd transparent. Hierdurch wird das Aufbringen des Gefässverschlusssystems als Punktionsverschluss an korrekter Position erleichtert. Ein transparenter Druckkörper lässt die Beobachtung der Punktionsstelle uneingeschränkt zu.

Vorzugsweise enthält das Gefässverschlusssystem ferner eine Aufnahme für einen Pulsfrequenzfühler.

Ausführungsbeispiele der Erfindung anhand der Zeichnung näher erläutert. Es zeigt:
- Fig. 1: eine Ansicht eines erfindungsgemässen aufklebbaren Gefässverschlusssystems;
- Fig. 2: eine Ansicht von unten auf das Gefässverschlusssystem gemäss Fig. 1;
- Fig. 3: einen Querschnitt des teilweise gezeigten Gefässverschlusssystems;
- Fig. 4: einen Längsschnitt durch eines nicht erfindungsgemässe Variante eines Gefässverschlusssystems; und
- Fig. 5: eine Ansicht von unten auf das Gefässverschlusssystem nach Fig. 4.

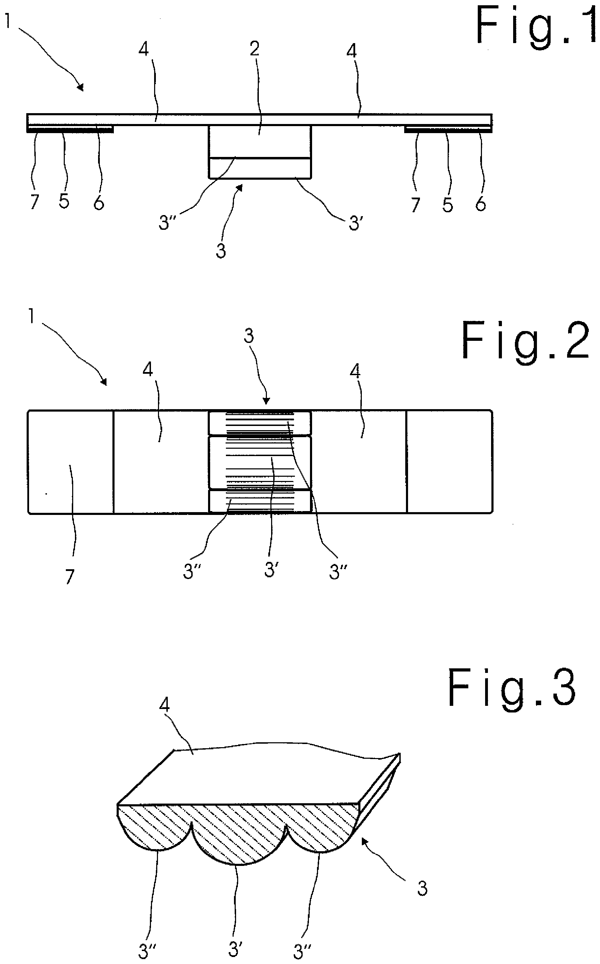

Fig. 1 und Fig. 2 zeigen ein aufklebbares Gefässverschlusssystem 1, welches ein Verschlusselement 2 und wenigstens einen Druckkörper 3 enthält, welche in diesem Beispiel einstückig aus einem Material mit elastischen Eigenschaften, beispielsweise Silikon, ausgebildet sind.

Der Druckkörper 3 ist erfindungsgemäss derart geformt, dass er im angelegten Zustand an einem geöffneten Gefäss bei einem Patienten einen erhöhten Druck erzeugt. Mit anderen Worten legt der Druckkörper 3 einen in Relation zur Einstichstelle im Gefäss reduzierten Druck an eine Punktionsstelle an der Haut und einen in Relation zur Einstichstelle im Gefäss reduzierten Druck an einem Bereich zur Pulsmessung am Gefäss an.

Der Druckkörper 3 ist als anatomische Form von drei Fingern gebildet. Hierbei steht die mittlere Fingernachbildung der insgesamt drei Fingernachbildungen in Relation zu den beidseitig anliegenden Fingern vor. Insgesamt ist die anatomische Form der Fingernachbildungen in Bezug auf eine Einstichstelle im Gefäss, auf eine Punktionsstelle an der Haut bzw. auf einen Bereich der Pulsmessung an dem Gefäss abgestimmt.

Zweckmässigerweise sind die drei gebildeten Druckkörper 3 mit je einer länglichen Druckfläche 3', 3" in Richtung der Haltebänder 4 angeordnet. Die beiden äusseren Druckflächen 3" sind dabei im Vergleich zu der mittleren Druckfläche 3' in der Höhe etwas zurückversetzt. Damit ergibt sich eine optimale Anpressung des Verschlusses an der Einstichstelle des Gefässes.

Ferner sind zwei die beiden Enden des Verschlusselements 2 bildenden Haltebänder 4 vorgesehen. Die Haltebänder 4 haben unelastische Eigenschaften. Beispielsweise können die Haltebänder 4 und das Verschlusselement 2 einstückig ausgebildet sein. Um den Haltebändern 4 die unelastischen Eigenschaften zu vermitteln, ist eine Verstärkungsschicht 5 auf den Haltebändern 4 angebracht. Die Verstärkungsschicht 5 kann mittels eines herkömmlichen Klebemittels 6 auf die Haltebänder 4 aufgeklebt werden. Um den dauerhaften Kontakt von Silikon mit dem verwendeten Klebemittel 6 zu verbessern, kann deshalb eine Oberflächenbehandlung, wie z.B. eine Plasma-, Corona-, nasschemische- oder sonstige Behandlung, vorgesehen sein.

Alternativ kann das gemeinsame Material, aus welchem das Verschlusselement 2, der Druckkörper 3 und die Haltebänder 4 einstückig ausgebildet sind, derart durch gezielte Aushärte-Verarbeitungsabläufe bearbeitet werden, dass lediglich nur das Material im Bereich der Haltebänder 4 keine Dehnung zulässt, während die restlichen Bereiche weiterhin dehnbar sind.

Im Prinzip können die Haltebänder 4 und das Verschlusselement 2 aus unterschiedlichen Materialien bestehen, welche miteinander verbunden sind. Dabei kann das Verschlusselement 2 aus Silikon und die Haltebänder 4 aus Polyethylen (PE) bestehen.

An der Unterseite der Verstärkungsschicht 5 ist eine Hautkleberschicht 7 zum Aufkleben auf die Haut bereitgestellt. Die Hautkleberschicht 7 hat vorzugsweise hautkleberspezifische Eigenschaften. Schliesslich kann die Hautkleberschicht 7 zusätzlich noch mit einer abziehbaren Schutzfolie versehen sein (nicht dargestellt).

Das Verschlusselement 2, der wenigstens eine anatomisch nachgebildete Druckkörper 3, die Haltebänder 4 und die Verstärkungsschicht 5 sind vorzugsweise transparent oder annähernd transparent ausgebildet, um das Aufbringen des ortsdefiniert aufklebbaren Gefässverschlusssystems 1 zu erleichtern.

Neben der ausserordentlichen Dehnbarkeit, weist Silikon auch eine erwünschte hohe Rückstellkraft auf. Es können aber auch andere Materialien, wie z.B. Naturkautschuk, Kunstkautschuk, Gummi, Latex, Hydrogel, polymere Kunststoff oder eine Kombination dieser Materialien, verwendet werden. Alternativ können die Haltebänder aus Polyethylen (PE) hergestellt sein.

Der anatomisch nachgebildete Druckkörper 3 hat im Normalfall (beispielsweise nach einer Untersuchung und/oder Behandlung, usw.) direkten Hautkontakt. Deshalb sind für die verwendeten Materialien geeignete medizinische Eigenschaften, vor allem hinsichtlich der Haut- und Körperverträglichkeit, erforderlich.

Bevor eine Nadel oder Kanüle 9 aus der Einstichstelle entnommen wird, wird die entsprechende Körperstelle auf herkömmliche Weise gereinigt und desinfiziert. Anschliessend werden die Schutzfolien von beiden Haltebändern des Gefässverschlusssystems 1 abgezogen. Das Gefässverschlusssystem 1 wird dann auf die betreffende Körperstelle mittels der Haltebänder derart angelegt und fixiert, dass der Druckkörper 3 später bei der vollständigen Applikation des Gefässverschlusssystems 1 derart zum Liegen kommt, dass ein maximaler Druck an der Einstichstelle des Gefässes angelegt wird. Zudem kommt der Druckkörper 3 derart zum Liegen, dass ein erhöhter Druck an der Punktionsstelle an der Haut des Patienten angelegt wird.

Ferner wird ein erhöhter Druck an einen Bereich zur Pulsmessung am Gefäss angelegt. Hierbei ist zu beachten, dass die beiden zuletzt genannten Drücke geringer sind als der Druck, welcher an der Einstichstelle des Gefässes angelegt wird. Hierdurch wird, analog zum medizinisch fachgemässen manuellen Abdrücken eines eröffneten Gefässes, die Einstichstelle des Gefässes maximal abgedrückt, während, in Relation hierzu, die Punktionsstelle an der Haut reduziert abgedrückt wird, um somit die ungehinderte Blutzufuhr zu gewährleisten. Anschliessend wird die Nadel, Kanüle 9 Schleuse oder Katheter entnommen.

Als Einsatzgebiete für das Gefässverschlusssystem 1 seien genannt: Gefässverschlusssystem zum Verschliessen von Gefässen nach interventionellen Eingriffen mit Kathetern über die Radialis, Femoralis, Cubitalis oder Brachialis; in der Allgemeinmedizin vor dem Entfernen von Verweilkanülen; in der Gefässchirurgie nach dem Entfernen von Varizen; nach minimalinvasiven chirurgischen Eingriffen vor dem Entfernen von medizinischen Gerätschaften.

Bei der genannten Anwendung des erfindungsgemässen Gefässverschlusssystems als Verschlusssystem zum Verschliessen von Gefässen, wird das Gefässverschlusssystem z.B. im Anschluss an eine Koronarintervention angelegt. Koronarinterventionen werden fast ausschliesslich über die Radialis oder die Femoralis durchgeführt. Arterielle Verschlusssysteme werden mit dem Ziel entwickelt, die Hämostasezeit zu verkürzen und gleichzeitig periphere Gefässkomplikationen zu vermeiden bzw. zu reduzieren. Eine im Vergleich zum Stand der Technik weiter verkürzte Hämostasezeit, bei gleichzeitiger Verhinderung peripherer Gefässkomplikationen, ist durch das erfindungsgemässe Gefässverschlusssystem erzielt.

Durch ein Strecken des Gefässverschlusssystems 1 durch Anlegen von jeweils einer Zugkraft an die Haltebänder 4 wird aufgrund der elastischen Eigenschaft des Materials des Verschlusselements 2 lediglich dieses in die Länge gestreckt.

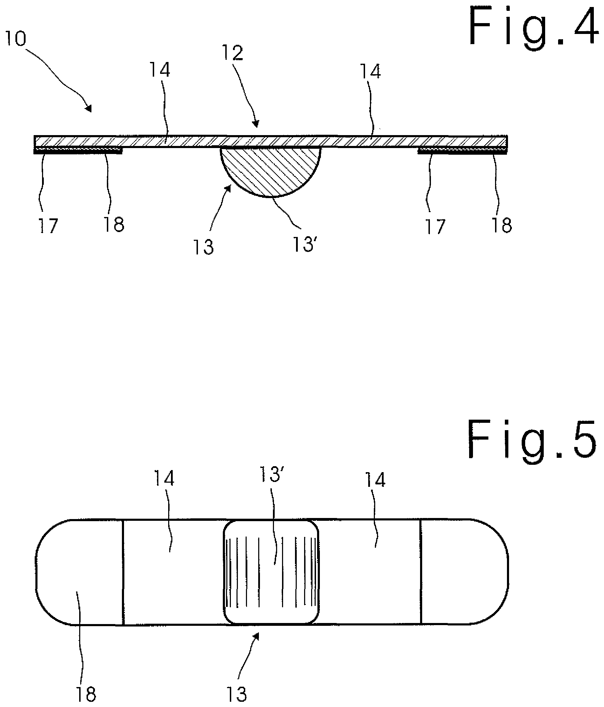

Fig. 4 und Fig. 5 zeigen eine nicht erfindungsgemässe Variante eines Gefässverschlusssystems 10 mit einem elastischen Verschlusselement 12 und einem Druckkörper 13. Letzterer weist eine Druckfläche 13' auf, welche beim Anlegen im Wesentlichen in Berührung mit der Haut gelangt. Der Druckkörper 13 weist eine längliche gerade Druckfläche 13' auf, die quer zur Längserstreckung der Haltebänder 14 verläuft. Sehr vorteilhaft ist diese Druckfläche 13' annähernd über die gesamte Breite und im rechten Winkel zu den Haltebändern 14 angeordnet. Dieser Druckkörper 13 ist in der Form eines Fingergliedes nachgebildet und ist als separater Teil auf den Haltebändern 14 beispielsweise aufgeklebt. Dies ergibt eine kostengünstige Herstellung des Verschlusses.

Damit kann im angelegten Zustand an einem geöffneten Gefäss bei einem Patienten einen in Relation zu weiteren Bereichen des Gefässes erhöhten Druck an eine Einstichstelle im Gefäss erzielt werden.

Ferner ist an der Unterseite der Haltebändern 14 zumindest eine Hautkleberschicht 17 zum Aufkleben auf die Haut sowie eine abziehbare Schutzfolie 18 vorhanden.

## Patentansprüche

1. Gefässverschlusssystem, mit Haltebändern (4) und einem Verschlusselement (2) mit wenigstens einem eine Druckfläche (3', 13') aufweisenden Druckkörper (3), wobei die Haltebänder (4) ein Material mit elastischen Eigenschaften und an der Unterseite zumindest eine Hautkleberschicht (17) zum Aufkleben aufweisen, wobei der Druckkörper (3, 13) so ausgelegt ist, dass im angelegten Zustand an einem geöffneten Gefäss bei einem Patienten einen in Relation zu weiteren Bereichen des Gefässes erhöhten Druck an eine Einstichstelle im Gefäss erzielt werden kann, **dadurch gekennzeichnet, dass**
der Druckkörper (3) als anatomische Form von drei Fingern mit je einer länglichen Druckfläche (3', 3") in Richtung der Haltebänder ausgebildet ist, wobei die beiden äusseren Druckflächen (3") im Vergleich zu der mittleren Druckfläche (3') in der Höhe etwas zurückversetzt sind.

2. Gefässverschlusssystem nach Anspruch 1, **dadurch gekennzeichnet, dass** der wenigstens eine Druckkörper (13) im rechten Winkel zur Längserstreckung der Haltebänder (14) und annähernd über die gesamte Breite dieser Haltebänder (14) angeordnet ist.

3. Gefässverschlusssystem nach Anspruch 1 oder 2, **dadurch gekennzeichnet, dass** der mittlere Finger sowie die beidseitig zu diesem benachbarten Fingern beim Druckkörper (3) nachgebildet sind.

4. Gefässverschlusssystem nach einem der vorhergehenden Ansprüche 1 bis 3, **gekennzeichnet durch** eine Vielzahl von Haltebändern (4), welche am Verschlusselement (2) anschliessen, wobei die Haltebänder (4) im Endbereich unelastische Eigenschaften aufweisen.

5. Gefässverschlusssystem nach einem der vorhergehenden Ansprüche 1 bis 4, **dadurch gekennzeichnet, dass** das Verschlusselement (2) und die Haltebänder (4) einstückig oder mehrstückig ausgebildet sind.

6. Gefässverschlusssystem nach einem der vorhergehenden Ansprüche 1 bis 5, **dadurch gekennzeichnet, dass** jeweils im Endbereich der Haltebänder (4) eine Verstärkungsschicht (5) angebracht ist, welche aus einem Material mit annähernd unelastischen Eigenschaften ausgebildet ist.

7. Gefässverschlusssystem nach Anspruch 6, **dadurch gekennzeichnet, dass** die Verstärkungsschicht (5) mittels eines Klebemittels (6) jeweils an einer Seite mit dem Bereich der Haltebänder (4) verbunden ist und gegenüber mit einer Hautkleberschicht (7) bereitgestellt ist.

8. Gefässverschlusssystem nach Anspruch 7, **dadurch gekennzeichnet, dass** die Hautkleberschicht (7) mit einer abziehbaren Schutzfolie bedeckt ist.

9. Gefässverschlusssystem nach einem der vorhergehenden Ansprüche 1 bis 8, **dadurch gekennzeichnet, dass** das Verschlusselement (2) und/oder der wenigstens eine Druckkörper (3) und/oder die Haltebänder (4) aus Silikon, Naturkautschuk, Kunstkautschuk, Gummi, Latex, Hydrogel, polymerem Kunststoff oder einer Kombination dieser Materialien bestehen.

10. Gefässverschlusssystem nach einem der vorhergehenden Ansprüche 1 bis 9, **dadurch gekennzeichnet, dass** das Verschlusselement (2) und/oder der wenigstens eine Druckkörper (3) und/oder die Haltebänder (4) und/oder die Verstärkungsschicht (5) transparent oder annähernd transparent sind.

11. Gefässverschlusssystem nach einem der vorhergehenden Ansprüche 1 bis 10, **dadurch gekennzeichnet, dass** ein Pulsfrequenzfühler enthalten ist.

## Claims

1. A vessel closure system comprising holding bands (4) and a closure element (2) with at least one pressure body (3) that has a pressure surface (3', 13'), whereas the holding bands (4) comprising a material with elastic properties and at least one skin adhesive layer (17) on the lower side for affixing, whereas the pressure body (3, 13) is shaped such that when in the placed condition on an opened vessel of a patient, increased pressure can be achieved at a puncture site within the vessel in relation to other regions of the vessel, **characterised in that**
the pressure body (3) is shaped as anatomical form of three fingers each with an elongate pressure surface (3', 3") in direction of the holding bands, whereas the either outer pressure surfaces (3") are somewhat set back in the height in comparison to the middle pressure surface (3').

2. The vessel closure system according to Claim 1, **characterised in that** the at least one pressure body (13) is arranged at right angles to the longitudinal extension of the holding bands (14) and over almost the entire width of these holding bands (14).

3. The vessel closure system according to Claim 1 or 2, **characterised in that** the middle finger and the fingers adjacent to the latter on either side are replicated in the pressure body (3).

4. The vessel closure system according to any of the preceding Claims 1 to 3, **characterised by** a plurality of holding bands (4) which adjoin the closure element (2), whereas the holding bands (4) having non-elastic properties in the end region.

5. The vessel closure system according to any one of the preceding Claims 1 to 4, **characterised in that** the closure element (2) and the holding bands (4) are made in one piece or in a number of pieces.

6. The vessel closure system according to any one of the preceding Claims 1 to 5, **characterised in that** in the respective end region of the holding bands (4) a reinforcement layer (5) is applied which is made of a material with almost non-elastic properties.

7. The vessel closure system according to Claim 6, **characterised in that** the reinforcement layer (5) is connected to the region of the holding bands (4) on one side respectively by means of an adhesive (6) and is provided with a skin adhesive layer (7) opposite.

8. The vessel closure system according to Claim 7, **characterised in that** the skin adhesive layer (7) is covered with a removable protective film.

9. The vessel closure system according to any one of the preceding Claims 1 to 8, **characterised in that** the closure element (2) and/or the at least one pressure body (3) and/or the holding bands (4) are made of silicon, natural rubber, synthetic rubber, gum, latex, hydrogel, polymer plastic or a combination of these materials.

10. The vessel closure system according to any of the preceding Claims 1 to 9, **characterised in that** the closure element (2) and/or the at least one pressure body (3) and/or the holding bands (4) and/or the reinforcement layer (5) are transparent or almost transparent.

11. The vessel closure system according to any of the preceding Claims 1 to 10, **characterised in that** a pulse frequency sensor is included.

## Revendications

1. Système d'obturation d'un vaisseau, comprenant des rubans (4) de maintien et un élément (2) d'obturation, ayant au moins une pièce (3) d'application d'une pression, ayant une surface (3', 13') d'application d'une pression, les rubans (4) de maintien ayant une matière à propriétés élastiques et, sur la face inférieure, au moins une couche (17) de colle à la peau pour le collage, la pièce (3, 13) d'application d'une pression étant conçue de manière à pouvoir, à l'état appliqué sur un vaisseau ouvert chez un patient, atteindre une pression à un point de piqûre du vaisseau plus haute qu'en d'autres endroits du vaisseau, **caractérisé en ce que**
la pièce (3) d'application d'une pression est constituée en une forme anatomique de trois doigts ayant chacun une surface (3', 3") oblongue d'application d'une pression dans la direction des rubans de maintien, les deux surfaces (3") extérieures d'application d'une pression étant un peu rétrogradées en hauteur par rapport à la surface (3') médiane d'application d'une pression.

2. Système d'obturation d'un vaisseau suivant la revendication 1, **caractérisé en ce que** la au moins une pièce (13) d'application d'une pression fait un angle droit avec l'étendue longitudinale des rubans (14) de maintien et s'étend à peu près sur toute la largeur de ces rubans (14) de maintien.

3. Système d'obturation d'un vaisseau suivant la revendication 1 ou 2, **caractérisé en ce que** le médium ainsi que les doigts qui en sont voisins de part et d'autre sont imités dans la pièce (3) d'application d'une pression.

4. Système d'obturation d'un vaisseau suivant l'une des revendications précédentes 1 à 3, **caractérisé par** une pluralité (4) de ruban de maintien, qui se raccorde à l'élément (2) d'obturation, les rubans (4) de maintien ayant des propriétés inélastiques dans la partie d'extrémité.

5. Système d'obturation d'un vaisseau suivant l'une des revendications précédentes 1 à 4, **caractérisé en ce que** l'élément (2) d'obturation et les rubans (4) de maintien sont constitués d'une seule pièce ou en plusieurs pièces.

6. Système d'obturation d'un vaisseau suivant l'une des revendications précédentes 1 à 5, **caractérisé en ce que**, respectivement dans la partie d'extrémité des rubans (4) de maintien, est mise une couche (5) de renfort constituée en un matériau ayant des propriété à peu près inélastiques.

7. Système d'obturation d'un vaisseau suivant la revendication 6, **caractérisé en ce que** la couche (5) de renfort est reliée au moyen d'un agent (6) adhésif, respectivement d'un côté, à la partie des rubans (4) de maintien et en face est préparée en ayant une couche (7) de colle à la peau.

8. Système d'obturation d'un vaisseau suivant la revendication 7, **caractérisé en ce que** la couche (7) de colle à la peau est revêtue d'une feuille de protection pouvant être retirée.

9. Système d'obturation d'un vaisseau suivant l'une des revendications 1 à 8, **caractérisé en ce que** l'élément (2) d'obturation et/ou la au moins une pièce (3) d'application d'une pression et/ou les rubans (4) de maintien sont en silicone, en caoutchouc naturel, en caoutchouc synthétique, en gomme, en latex, en hydrogel, en matière plastique polymère ou en une combinaison de ces matières.

10. Système d'obturation d'un vaisseau suivant l'une des revendications précédentes 1 à 9, **caractérisé en ce que** l'élément (2) d'obturation et/ou la au moins une pièce (3) d'application d'une pression et/ou les rubans (4) de maintien et/ou la couche (5) de renfort sont transparents ou à peu près transparents.

11. Système d'obturation d'un vaisseau suivant l'une des revendications précédentes 1 à 10, **caractérisé en ce qu'**il contient un détecteur de la fréquence du pouls.
